# EUROPEAN PATENT APPLICATION

(11) **EP 3 906 981 A1**
(43) Date of publication of application: **10.11.2021**
(21) Application number: 20173586.7
(22) Date of filing: 07.05.2020
(51) Int. Cl.: B01D 11/02, A23F 3/36, A23F 5/20, A23G 1/00, A61K 36/00, B27K 7/00, C11B 9/02

(54) **METHOD FOR EXTRACTION USING CARBON DIOXIDE**

(71) Applicant: Feyecon Development & Implementation B.V., 1382 GS Weesp (NL)
(72) Inventor: de Vries, Tjerk Jan, 1382 GS Weesp (NL); Hofland, Gerard Willem, 1382 GS Weesp (NL); Woerlee, Geert Feye, 1382 GS Weesp (NL); Vale Lopes, Paulo Dinis, 1382 GS Weesp (NL)
(74) Representative: van Essen, Peter Augustinus

(57) **Abstract**

The invention provides a method for extraction of at least one extract compound from a material, comprising:
a. contacting carbon dioxide with the material to dissolve an amount of the at least one extract compound from the material into the carbon dioxide;
b. contacting the carbon dioxide comprising the at least dissolved extract compound with a sorbent to sorb the at least one extract compound onto the sorbent and for regenerating the carbon dioxide;
c. recirculating the regenerated carbon dioxide; and
d. repeating said recirculating at a constant density of said carbon dioxide of between 100 and 1000 kg/m³, and repeating said recirculating for at least 10 cycles.

## Description

### Field of the invention

The invention relates to a method for extraction of at least one extract compound from a material.

### Background of the invention

Many materials from agriculture and biobased processing are interesting for food, cosmetics or technical applications, if it where not that they contain by low levels of components that are for instance adding a bad taste, are anti-nutritional, are pharmaceutically active, are hazardous or even toxic. Conversely, such components could be valuable in their own right but are considering their low levels difficult to capture efficiently, at limited costs and without producing large effluent streams.

Removal by (organic) solvents is too hazardous, prevents application of the material in food and other products that come in contact with people, etc. Moreover, removal of compounds from these solvents is expensive and hazardous.

Using supercritical CO₂ as a solvent has been proposed for many applications, but still is considered expensive in terms of equipment cost, due to it high pressure, and operating costs, due to the often moderate solubilities, the associated large volumes that need to be pumped to high pressures.

WO2005025825, entitled "Method for the Extraction of Cork-Containing Material", discloses a method for extracting cork-containing material with the aid of a compressed gas at temperatures ranging between 10 and 120 DEG C and pressures ranging between 10 and 600 bar. Extraction takes place in isobaric conditions while the compressed gas flows through the charge in a radial or axial direction and the charge that is to be extracted is combined with an adsorbing material. The inventive method allows cork plates, cork granules, bottle cork, or agglomerated cork, for example, to be quantitative removed from organic compounds such as pentachlorophenol, trichloroanisole, and tetrachloroanisole as well as waxes and fats such that the purified cork-containing materials can be used without reservation in the food sector and particularly in the beverage sector.

Many known processes require additional steps like filtering, that require changing process parameters like pressure of temperature of the carbon dioxide.

Although the above-mentioned processes may be more or less effective as to the removal of contaminants from cork, they all suffer from the drawback of needing complex systems to be implemented. At an industrial scale, such complexity implies great-deals of equipment, energy and maintenance costs.

On the other hand, the compression-decompression cycles of the conventional treatment continuously dry the cork material along the cycle operation time, with serious consequences for the structural integrity thereof. In fact, the imposed pressure drop may decrease the water solubility in the extraction fluid, giving rise to its condensation in the collection vessel. In this way, the water that is transferred from cork material to the extraction fluid (cork drying) is subsequently eliminated in the collection vessel by condensation.

Also, the particular removal of large molecules compounds, like long-chain aliphatic alcohols, waxes and fats, from the extraction fluid requires dedicated equipment and specific thermodynamic conditions so as to reduce the solubility of the extraction fluid and therefore to precipitate said large molecules compounds, thus turning the process and respective systems complex to implement and maintain.

The present invention is therefore directed at a method of extracting at least one compound from a material, an assembly for extracting, and a plant material, which overcome one or more of the disadvantages of the prior art, without jeopardizing other characteristics.

### Summary of the invention

We now present a method to easily extract compounds from material to valorise these in an economical manner, without large solvent streams. In a specific embodiment, the extracted compound is easily retrieved as a concentrate from the process, for the case that its retrieval is important to minimize waste or that it in itself is a valuable product, e.g. a pharmaceutically acceptable product. The present method provides a cost effective way of using carbon dioxide extraction, and provides low amounts of harmful (extracted) compounds in the treated material, typically less than a few ppm, and sometimes much lower. The extraction can be done with minimally affecting nutritional, flavour and mouthfeel aspects and retrieving such compounds as valuable concentrates for pharmaceutical or other applications.

The present method for extraction of at least one extract compound from a material, comprises contacting carbon dioxide with the material to dissolve an amount of the at least one extract compound from the material into the carbon dioxide; contacting the carbon dioxide comprising the at least dissolved extract compound with a sorbent to sorb the at least one extract compound onto the sorbent and for regenerating the carbon dioxide; recirculating the regenerated carbon dioxide; and repeating said recirculating at a density of said carbon dioxide of between 100 and 1000 kg/m³, and repeating said recirculating for at least 10 cycles, such as for at least 20 cycles.

In an second aspect the present invention relates to an assembly for extraction of at least one extract compound from a material, comprising a pressure vessel enclosing: a pump in said pressure vessel for circulating carbon dioxide through said pressure vessel; a sorbent container in said pressure vessel for holding a sorbent, and a material container in said pressure vessel for holding the material comprising the at least one extract compound, said pump fluidly coupled to said material container and to said sorbent container for circulating said carbon dioxide through said material and through said sorbent at a density of between 100 and 1000 kg/m³.

In a third aspect the present invention relates to an extract plant material comprising less than 1 ppm pesticide, preferably less than 10 ppb pesticide.

Said plant material may be obtainable by the present method or in the present assembly. By removing harmful compounds, through extraction, very otherwise pure materials can be obtained, which are suited for further use, and which in some examples could be used as a pharmaceutical ingredient, such as in a medicament as active compound.

Hence, it is an aspect of the invention to provides an improved process, which preferably further at least partly obviates one or more of above-described drawbacks.

### DETAILED DESCRIPTION OF THE INVENTION

In a first aspect the present invention relates to a method of extracting at least one compound.

In an exemplary embodiment of the present method during said recirculating said carbon dioxide may be maintained at a substantially constant pressure, preferably a pressure drop for one cycle is less than 1 MPa (10 bar), preferably less than 0.5 MPa (5 bar), more preferably less than 0.1 MPa (1 bar).

In an exemplary embodiment of the present method said constant pressure may be between 2 and 50 MPa (20-500 Bar), more preferably between 4 and 20 MPa (40-200 bar).

In an exemplary embodiment of the present method during said recirculating said carbon dioxide maybe maintained at a substantially constant temperature, preferably said temperature is maintained within a range of less than ±20 degrees Celsius, more preferably said temperature is maintained within a range of less ±10 degrees Celsius, more preferably less than ±1 degree Celsius.

Reducing fluctuations in the process can reduce energy consumption and can prevent condensation. It can further allow a process that does not require cyclone filtering, for instance.

In an exemplary embodiment of the present method said constant temperature may be between 10 and 140 °C, more preferably between 20 and 100 °C, more preferably between 40 and 70 °C.

In an exemplary embodiment of the present method said sorbent may be selected from the group consisting of activated carbon, silicates, natural and synthetic mineral clays, aluminosilicates, phyllosilicates, aluminium phyllosilicates, zeolite material, maltodextrins, super absorbing polymers, and a combination thereof. These sorbents are considered to function mainly as chemical sorbents, i.e. through chemisorption, rather than through adsorption or physisorption. Chemisorption is a kind of sorption which involves a chemical reaction between the surface of the sorbent material and the adsorbate, in this case the at least one extracted compound. New chemical bonds are generated at the sorbent surface. Upon forming new bonds typically an activation energy is involved. Also chemisorption typically improves with increasing temperatures. Especially clay minerals, zeolites, and the like, having a large surface, function well. In particular sorbents with a specific surface of > 100 m²/gr sorbent function well; such a specific surface can be measured using e.g. Brunauer-Emmett-Teller (BET) techniques. In alternative embodiments, the sorbent may comprise natural polymers. Examples are cellulose material. Alternatively or in combination, the sorbent comprises synthetic polymers.

In an exemplary embodiment of the present method a weight ratio of said carbon dioxide to material may be between 10:1 and 1:1, preferably between 5:1 and 1:1.

In an exemplary embodiment of the present method the material may be a plant material, preferably wherein the plant material before extraction comprises 1% or less extract compound, and/or wherein a ratio of material to sorbent is between 5:1 and 1000:1, preferably between 20:1 and 200:1.

In an exemplary embodiment of the present method the carbon dioxide may comprise between 10 and 100% of the maximum solubility of water in carbon dioxide at said constant density and temperature. In an embodiment, the carbon dioxide may comprise between 50 and 95% of the maximum solubility of water in carbon dioxide at said constant density and temperature. In yet a further embodiment, the carbon dioxide may comprise more preferably between 60 and 90 % w/w of the maximum solubility of water in carbon dioxide at said constant density and temperature.

In an exemplary embodiment of the present method the material may be a plant material selected from tree bark, root, needles, beans, pods, seeds, hard fruit, flowers, woods, and mixtures thereof. In an embodiment, the beans are selected from coffee beans, cocoa beans, soy beans, and peas. In an embodiment, the plant material comprises pre-processed plant material. In this respect, pre-processing can comprise milling, crushing, cutting, heat-treating, steam processing, drying, soaking, and combinations of these steps.

In an exemplary embodiment of the present method the at least one extract compound may be selected from at least one polycyclic aromatic compound, a halogenated compound, a terpenoid, a cannabinoid, a triterpenoids, a pesticide, a converted pesticide and combination thereof. In an embodiment, the converted pesticide is selected from a microbially, enzymatically, (photo) chemically, and incompletely combusted pesticide.

In an exemplary embodiment of the present method the material may be selected from Eucalyptus, birch bark, and mixtures thereof, and wherein the at least one extract compounds comprises at least one triterpenoid acid.

In an exemplary embodiment of the present method the at least one extract compound may comprise a at least one polycyclic aromatic compound.

In an exemplary embodiment of the present assembly at least one of the sorbent container and the material container each comprise a permeable wall allowing said carbon dioxide to respectively at least one of entering the respective sorbent container, entering the material container, exiting the sorbent container, exiting the material container, and a combination thereof.

In an exemplary embodiment of the present assembly said sorbent container and said material container are fluidly coupled in series to one another, in particular wherein said actuator, said sorbent container and said material container are fluidly coupled in series to one another.

The extract compound in an embodiment may comprise one or more diterpenes, such as taxanes. Taxanes present difficulties in formulation as medicines because they are poorly soluble in water. Taxanes are alkaloids possessing a taxane nucleus. In general, a taxane nucleus comprises the three ring structure shown below which is also identified as 4,8,12,15, 15-pentamethyl-tricyclo (9.3.1.O] pentadecane (CAS 1605-68-1).

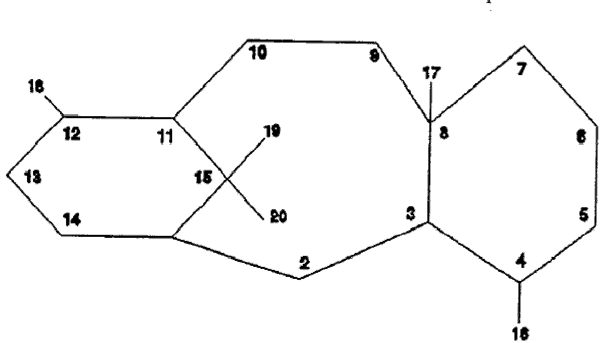

Among the taxane molecules which have been studied most with respect to for instance their antitumor activity are taxol, cephalomannine, desacetylcephalomannine, baccatin III, 10-desacetyl baccatin III and 10-desacetyltaxol. It is noted that mostly these diterpenes are at the same time highly toxic. The structures of these taxanes are shown below:

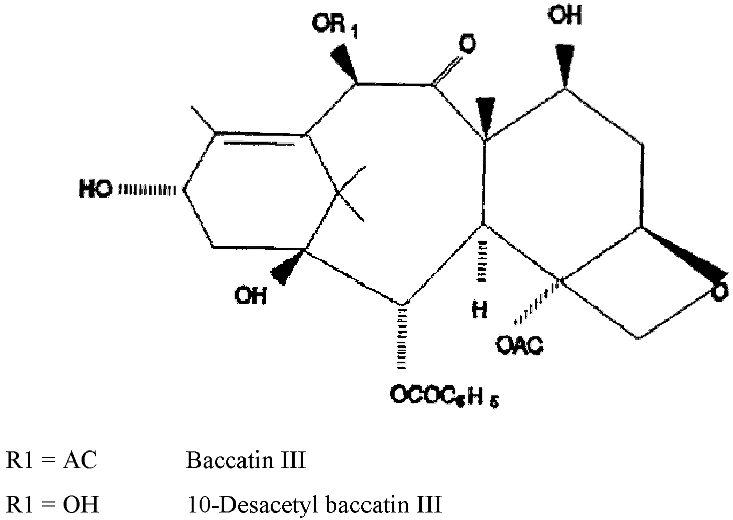

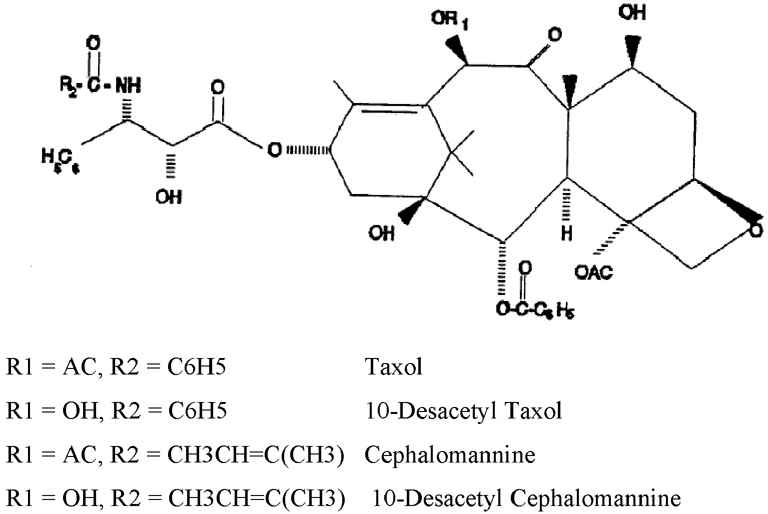

The terms "upstream" and "downstream" relate to an arrangement of items or features relative to the flow of fluid from a pump.

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of'.

The term "functionally" will be understood by, and be clear to, a person skilled in the art. The term "substantially" as well as "functionally" may also include embodiments with "entirely", "completely", "all", etc. Hence, in embodiments the adjective functionally may also be removed. When used, for instance in "functionally parallel", a skilled person will understand that the adjective "functionally" includes the term substantially as explained above. Functionally in particular is to be understood to include a configuration of features that allows these features to function as if the adjective "functionally" was not present. The term "functionally" is intended to cover variations in the feature to which it refers, and which variations are such that in the functional use of the feature, possibly in combination with other features it relates to in the invention, that combination of features is able to operate or function. For instance, if an antenna is functionally coupled or functionally connected to a communication device, received electromagnetic signals that are receives by the antenna can be used by the communication device. The word "functionally" as for instance used in "functionally parallel" is used to cover exactly parallel, but also the embodiments that are covered by the word "substantially" explained above. For instance, "functionally parallel" relates to embodiments that in operation function as if the parts are for instance parallel. This covers embodiments for which it is clear to a skilled person that it operates within its intended field of use as if it were parallel.

Furthermore, the terms first, second, third and the like when used in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices or apparatus herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements.

The mere fact that certain measures are recited in mutually different dependent claims does not indicate that a combination of these measures cannot be used to advantage.

The invention further applies to an apparatus or device comprising one or more of the characterising features described in the description and/or shown in the attached drawings. The invention further pertains to a method or process comprising one or more of the characterising features described in the description and/or shown in the attached drawings.

The various aspects discussed in this patent can be combined in order to provide additional advantages. Furthermore, some of the features can form the basis for one or more divisional applications.

### Brief description of the drawings

Embodiments of the invention will now be described, by way of example only, with reference to the accompanying schematic drawings in which corresponding reference symbols indicate corresponding parts, and in which:
Figure 1 schematically depicts an embodiment of the assembly;
Figure 2 a graph showing a relation between maximum solubility of water in carbon dioxide.

The drawings are not necessarily on scale.

### Description of preferred embodiments

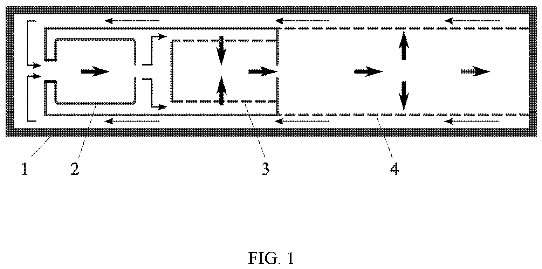

In figure 1, an example of the assembly is schematically depicted. A pressure vessel 1 holds a pump 2, and a first containers 3 and a second container 4. The pump 2 and the containers are mutually coupled in series. One of the containers 3, 4 can hold the material, and the other container 4, 3 can hold the sorbent. In an embodiment, more than one sorbent can be used for sorbing different extract compounds. In such an embodiment, a container can be divided into subcontainers each holding a separate sorbent.

In this embodiment, the pump is fluidly coupled with first container 3. The first container is in turn fluidly coupled with the second container 4. A space between an inner surface of a wall of the pressure vessel 1 and an outer surface of at least one of the first and second container 3, 4 can provide a conduit for a return flow of carbon dioxide, fluidly coupling the second container 4 and an inlet of the pump 2.

In an embodiment, the pressure vessel 1 is circle cylindrical. In a further embodiment, the first and second container 3, 4 and circle cylindrical. In depicted embodiment, flows of carbon dioxide are indicated with arrows, showing a flow direction. In the embodiment, each container 3, 4 has a radial flow opening and an axial flow opening. In the depicted embodiment, the first container comprises a radial ingress provision, here a permeable circumferential wall. The first container comprises an axial outlet opening. In an embodiment, a permeable conduit may be provided. In an embodiment, the inlet opening may be fluidly coupled to the permeable, axial conduit and the outlet opening may be provided by the permeable circumferential wall.

In the depicted embodiment, the second container has its inlet opening be fluidly coupled to a permeable, axial conduit and its outlet opening may be provided by the permeable circumferential wall. For a compact build, the first and second containers have reverse inlet and outlet provisions.

At least part of one or more walls of the first and/or second container are permeable to carbon dioxide. Here, the circumferential walls of both first and second container are permeable. The walls may comprise for instance comprise a perforated part, a sieve part. In an embodiment, one or more parts of one or more container wall parts may comprise a cloth, nonwoven part, or a mesh. For the permeable circumferential wall and/or axial permeable conduit, in order to provide a homogeneous flow, the permeability in axial direction may increase.

Other configurations may be possible. The depicted embodiment or the alternative with first and second containers with mutually reverse inlet and outlet provisions allow a compact build.

Pump 2 can have a capacity and limited headspace for providing a pressure increase of less than 5 bar.

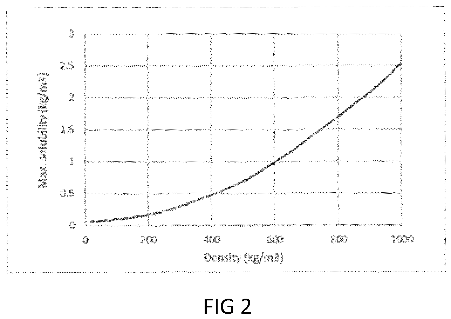

Figure 2 shows a relation between water solubility of water in carbon dioxide an the density of the carbon dioxide. In this graph, the temperature of carbon dioxide is 40 °C.

### Examples

### 1. Cleaning of cork

Unprocessed cork (200 g) was cut to granules with size of about mm, having 11.8 ppt (parts per trillion!) TCA and a moisture content of ca. 10%. These cork dices were placed in a 600 ml canister in a vertically placed 2 litre stainless steel pressure vessel. This canister was made from stainless steel with a two layer mesh-perforated plate top and bottom parts. A second, bag-like, canister was placed in the same pressure vessel and on top of the first canister. The second canister contained 100 g. of active carbon.

Carbon dioxide was introduced in the system to a pressure of 140 bar and a temperature 40°C. The carbon dioxide was subsequently circulated from top to bottom over the two canisters for 1 hour, using a centrifugal pump at a max. pressure drop of 0.8 bar and a flow rate of 126 kg/h. TCA contents were determined after depressurization of the vessel in both the cork and active carbon. TCA contents were measured after extraction by SPME-GC-MS according to International Standard ISO 20752.

In a second trial, two sets of first and second canisters were placed into the pressure vessel and the process was continued for 1.5 h.

In both runs, TCA level in the cork was reduced to below 0.5 ppt (parts per trillion).

### 2. Cleaning of cocoa beans

Cocoa beans may have contaminants such as fungi, pesticides, heavy metals and PAHs on their shell. These contaminants may get into the chocolate production process and contaminate the final chocolate product, which is unhealthy for consumers. Supercritical CO₂ was used to clean the cocoa beans and remove these contaminants from the cocoa bean shell.

To remove the contaminants from the cocoa beans a drying equipment was used, which is a system which circulates supercritical CO₂ through two pressure vessels. These two pressure vessels can be brought into a fluid communication.

Some options for the configuration of the two vessels, where the far right one is the preferred version. The left one was used in this example. Supply and discharge of supercritical CO₂ is not shown here.

In the larger vessel of these two vessels, a measured amount of cocoa beans was put in a cloth bag functioning as a canister, to prevent beans or parts/dust of the bean to enter and circulate through the system. In the smaller of the two vessels, an excess of active carbon was placed in a cloth holder, again to prevent dust to circulate through the system. The system was pressurised, and the CO₂ was circulated through the bags functioning as beds and the system comprising the two pressure vessels for 1 hour at a flow rate of 237 kg/h and a maximum pressure drop of 0.8 bar. The temperature of the carbon dioxide in the system was 40°C and the pressure was 150 bars.

With this method very little water or fat is extracted from the beans and there is a significant decrease in pesticides and polyaromatic hydrocarbons, as the table below illustrates.

| Table | Before | After |
|---|---|---|
| Pyrethrins (sum) | 2.43 mg/kg | ND |
| Fenvalerate (sum) | 1.81 mg/kg | 0.4 mg/kg |
| Sample weight | 100.2g | 99.6g |
| Active carbon weight | 10.0 g | 10.1g |

The pyrethrins are a class of organic compounds normally derived from Chrysanthemum cinerariifolium that have potent insecticidal activity by targeting the nervous systems of insects. Pyrethrin naturally occurs in chrysanthemum flowers and is often considered an organic insecticide.
Fenvalerate (CAS 51630-58-1) is a pyrethroid insecticide. It is a mixture of four optical isomers which have different insecticidal activities. The 2-S alpha (or SS) configuration, known as esfenvalerate, is the most insecticidally active isomer. Fenvalerate consists of about 23% of this isomer.

### 3. Taxane removal from hazelnut shells

Hazelnut shells contain taxane derivatives (Miele et al., Phytochem. Rev. 11: 211-225 (2012) that may limit new applications (Ref: Luisa Cruz-Lopes, Jorge Manuel Martins, Bruno Esteves, B. Lemos. ECOWOOD 2012 conference 5th International Conference on Environmentally-Compatible Forest Products. 05-7 September 2012 Oporto, Portugal) by the presence of taxanes (paclitaxel derivatives/precursors), which by itself could be relevant pharmaceutical products or building blocks.

Hazelnut in shell were obtained from a regional wholesaler and dehulled. Shells were dried for 24 h before further use. Shells further crushed to sizes of ca. 3-5 mm, and further milled in a blender and mortar. A 92 g of the crushed shells was packed in a cloth container and placed in a cylindrical high pressure container of 1 litre, together with 9 g of active carbon (Cabot, granules) placed on top. After adding 5 ml of ethanol as co-solvent/entrainer, precooled CO₂ was pump using a Lewa membrane pump type EK1 and heated till 40°C by a tube-in-tube heater exchanger and added to the high pressure container till a pressure of 160 bar was reached. CO₂ was recirculated at a rate of 100 kg/h over the two beds (top-to-bottom) via and internal high static pressure centrifugation pump for 1 hour, which keeping the temperature of the high pressure container maintained at 40°C through a hot water hose jacketed around the container. The overall pressure drop was measured using a pressure difference meter to be 35 mbar over the system.

Pressure was released during 7 min from a bottom connection of the vertically placed container into a second container where the residuals were retrieved at the bottom.

Initial and residual contents as well as the yield in active carbon was determined for taxane derivative (baccatin III) via HPLC-UV at 225 nm after 20µl injection on a reversed phase column (Phenomenex Luna, 250x4.6 mm, 5µm) eluted with water:methanol: acetonitril 50:30:20.

**Table**

| | |
|---|---|
| Initial taxane content in the shells | 35 mg/kg |
| Final taxane content in the shells | 6 mg/kg |
| Taxane content in active carbon | 2.2 mg |

Results in the table above show that the levels in the shell was significantly reduced and the taxane was largely captured on the active carbon in concentrated form, allowing the CO₂ to be safely released, the shell to be safely used and the pharmaceutically active component to be further used.

### 4. Extraction of rose bud oil

Freshly harvested rose bud petals were treated to obtain rose oil which is present in low level amounts. A 600 ml canister was filled with 90 g of petals. A separate bag was filled with 10 g fine powdered hardened rapeseed RP70 (Cargill). Both were brought into a high pressure vessel, which was kept at 25°C and subsequently pressurised to 70 bars using dense carbon dioxide. Carbon dioxide was circulated over the two beds placed in series, flow rate 100 kg/h, for 1.5 h. After circulation the system was depressurised during 10 minutes. In this process, the water content was close to 100% of the water of the maximum solubility of water in carbon dioxide at the given temperature and pressure.

Processed Rose petals and RP70 were subsequently extracted with acetone for 5 days at -18°C for analysis. Rose oil contents were determined in duplicate on the base of its principle component, citronellol, using GC Varian 430-GC equipped with a Agilent VF-1ms column and FID detector. See the results in the table below, showing amounts in rose petals before extraction, after extraction ("processed") and amounts in rapeseed fat. Thus, rose oil is obtained in a commercially viable method, that is selective.

| | Citronellol content (mg/g) |
|---|---|
| Rose petals | 0.34 +/- 0.04 |
| Processed rose petals | 0.06 +/- 0.03 |
| Fat | 2.53 +/- 0.07 |

It will also be clear that the above description and drawings are included to illustrate some embodiments of the invention, and not to limit the scope of protection. Starting from this disclosure, many more embodiments will be evident to a skilled person. These embodiments are within the scope of protection and the essence of this invention and are obvious combinations of prior art techniques and the disclosure of this patent.

## Claims

1. Method for extraction of at least one extract compound from a material, comprising:
a. contacting carbon dioxide with the material to dissolve an amount of the at least one extract compound from the material into the carbon dioxide;
b. contacting the carbon dioxide comprising the at least dissolved extract compound with a sorbent to sorb the at least one extract compound onto the sorbent and for regenerating the carbon dioxide;
c. recirculating the regenerated carbon dioxide; and
d. repeating said recirculating at a constant density of said carbon dioxide of between 100 and 1000 kg/m³, and repeating said recirculating for at least 10 cycles.

2. Method according to claim 1, wherein during said recirculating said carbon dioxide has a pressure drop for one cycle less than 1 MPa (10 bar), preferably less than 0.5 MPa (5 bar), more preferably less than 0.1 MPa (1 bar).

3. Method according to claim 1, 2 or 3, wherein during said recirculating said carbon dioxide is maintained at a substantially constant temperature, preferably said temperature is maintained constant within a range of less than ±20 degrees, more preferably said temperature is maintained constant within a range of less ±10, more preferably less than ±1 degree.

4. Method according to claim any of the preceding claims, wherein said constant temperature is between 10 and 140 °C, preferably between 20 and 100 °C, more preferably between 40 and 70 °C.

5. Method according to claims 1 or 2, wherein said sorbent is selected from the group consisting of activated carbon, silicates, natural and synthetic mineral clays, aluminosilicates, phyllosilicates, aluminium phyllosilicates, zeolite material, maltodextrins, super absorbing polymers, and a combination thereof.

6. Method according to claims 1-3, wherein a weight ratio of said carbon dioxide to material of between 10:1 and 1:1.

7. Method according to claim any of the preceding claims, wherein the material is plant material, preferably wherein the plant material before extraction comprises 1% or less extract compound, and/or wherein a ratio of material to sorbent is between 5:1 and 1000:1, preferably between 20:1 and 200:1.

8. Method according to claim 4, wherein the carbon dioxide comprises between 10 and 100% of the maximum solubility of water in carbon dioxide at said constant density and temperature, preferably between 50 and 95% maximum solubility of water in carbon dioxide at said constant density and temperature.

9. The method of any one of the preceding claims, wherein the material is a plant material selected from tree bark, root, needles, beans, pods, seeds, hard fruit, flowers, woods, and mixtures thereof, wherein preferably the beans are selected from coffee beans, cocoa beans, soy beans, and peas, and/or wherein preferably the plant material comprises pre-processed plant material.

10. The method of any one of the preceding claims, wherein the at least one extract compound is selected from at least one polycyclic aromatic compound, a halogenated compound, a terpenoid, a cannabinoid, a triterpenoids, a pesticide, a converted pesticide and combination thereof, wherein preferably the converted pesticide is selected from a microbially, enzymatically, (photo) chemically, and incompletely combusted pesticide.

11. Assembly for extraction of at least one extract compound from a material, comprising a pressure vessel enclosing:
- a pump in said pressure vessel for circulating carbon dioxide through said pressure vessel;
- a sorbent container in said pressure vessel for holding a sorbent, and
- a material container in said pressure vessel for holding the material comprising the at least one extract compound,
said pump fluidly coupled to said material container and to said sorbent container for circulating said carbon dioxide through said material and through said sorbent at a density of between 100 and 1000 kg/m³.

12. The assembly of claim 11, wherein at least one of the sorbent container and the material container each comprises a permeable wall allowing said carbon dioxide to respectively at least one of entering the respective sorbent container, entering the material container, exiting the sorbent container, exiting the material container, and a combination thereof.

13. The assembly of claim 11 or 12, wherein said sorbent container and said material container are fluidly coupled in series to one another, in particular wherein said actuator, said sorbent container and said material container are fluidly coupled in series to one another.

14. The assembly of any one of the preceding claims 11-13, where the pump is adapted to overcome a pressure drop over the assembly of maximum 5 bar, preferably less than 1 bar.

15. Extract plant material comprising less than 1 ppm pesticide, preferably less than 10 ppb pesticide.
